# EUROPEAN PATENT APPLICATION

(11) **EP 4 202 048 A1**
(43) Date of publication of application: **28.06.2023**
(21) Application number: 21216354.7
(22) Date of filing: 21.12.2021
(51) Int. Cl.: C12N 15/86

(54) **ALPHARETROVIRUS-BASED PARTICLES FOR DELIVERY OF RNA INTO CELLS**

(71) Applicant: Medizinische Hochschule Hannover, 30625 Hannover (DE)
(72) Inventor: Schambach, Axel, 30625 Hannover (DE); Galla, Melanie, 30625 Hannover (DE); Baron, Yvonne, 30625 Hannover (DE)
(74) Representative: Taruttis, Stefan Georg

(57) **Abstract**

The new alpharetrovirus-based particles are suitable for high efficiency of transiently transducing animal cells, e.g. human or murine cells, and which efficiently introduce coding and non-coding RNA contained in the alpharetrovirus-based particles into target cells The alpharetrovirus-based particles also provide for high efficiency of the activity and/or integrity of the RNA that is introduced into the animal cells, as the particles protect the incoming RNA from degradation during entry. The transferred RNA can be of non-coding nature (e.g. single guide (sg) RNA, short-hairpin (sh) RNA, micro RNA, or long non-coding (lnc) RNA, or the RNA may encode proteins or peptides, e.g. receptors, transcription factors, cellular enzymes, antigens for use in vaccination, gene/protein therapy and/or gene editing nucleases, recombinases and transposases.

## Description

The present invention provides alpharetrovirus-based particles that are suitable for high efficiency of transiently transducing animal cells, e.g. human or murine cells, and which efficiently introduce coding and non-coding RNA contained in the alpharetrovirus-based particles into target cells The alpharetrovirus-based particles also provide for high efficiency of the activity and/or integrity of the RNA that is introduced into the animal cells, as the particles protect the incoming RNA from degradation during entry. The transferred RNA can be of non-coding nature (e.g. single guide (sg) RNA, short-hairpin (sh) RNA, micro RNA, or long non-coding (lnc) RNA, or the RNA may encode proteins or peptides, e.g. receptors, transcription factors, cellular enzymes, antigens for use in vaccination, gene/protein therapy and/or gene editing nucleases, recombinases and transposases. In addition, the transferred RNA can be a self-amplifying RNA replicon encoding any of the mentioned RNA classes. The alpha-retrovirus based particles may contain at least one, e.g. at least two or three RNA constructs, which may consist of coding or non-coding RNAs, or combinations of both. The gene editing nuclease can e.g. be selected from the transcription activator-like effector (TALEN), zinc-finger (ZFN), and, preferably, RNA-guided clustered regularly interspaced short palindromic repeats (CRISPR) / CRISPR-associated (Cas) nucleases, especially CRISPR/Cas9, also in combination with a sgRNA. The recombinase can e.g. selected from Cre, Dre, Tre, Brec, Flp recombinases, and transposases can be e.g. selected from Sleeping Beauty, PiggyBac transposons.

### State of the art

Knopp et al., Mol. Ther. Nucleic Acids 13, 256-274 (2018), "Transient retrovirus-based CRISPR/Cas9 All-in-One Particles for efficient, targeted gene knock-out", describes a gammaretrovirus-based particle delivering CRISPR/Cas9 RNA for genetically manipulating human and murine cells.

Hoffmann D. et al., Detailed comparison of retroviral vectors and promoter configurations for stable and high transgene expression in human induced pluripotent stem cells, Gene Ther. 2017 May;24(5):298-307 describes a reporter gene construct for EGFP (Vector name = RRL.PPT.EFS.EGFP.PRE).

Heckl D, Kowalczyk MS, Yudovich D, Belizaire R, Puram RV, McConkey ME, Thielke A, Aster JC, Regev A, Ebert BL. Generation of mouse models of myeloid malignancy with combinatorial genetic lesions using CRISPR-Cas9 genome editing. Nat Biotechnol. 2014 Sep;32(9):941-6, describes a vector containing RFP657.Tet2 reporter gene construct for Tet2. (Vector name = LKO5d.SFFV.tRFP657(Tet2.4).iPAC)

### Object of the invention

An object of the invention is to provide an alpharetrovirus-based particle that efficiently transduces animal cells, especially human cells, and efficiently delivers at least one RNA construct into recipient cells in a transient manner. The retrovirus-based particle can contain one or more RNA construct species of interest at the same time, which allows spatiotemporal co-delivery of the latter into target cells, e.g. for transiently expressing from the RNA construct, e.g. a gene editing nuclease alone or in combination with an RNA construct that is a non-coding RNA, e.g. a sgRNA and/or a transgene RNA construct for altering the target cell genome. Advantageously, the non-integrating alpharetrovirus-based particle shall contain an RNA construct that does not cause insertional mutagenesis, more preferably, the retrovirus-based particle shall deliver an RNA construct for transient activity only in recipient cells, thereby also avoiding or lowering the likelihood of cytotoxicity mediated by stable and prolonged overexpression of certain transgenes (e.g. SpCas9, Cre recombinase, ZFN nucleases, Sleeping Beauty DNA transposase) and/or other RNA species.

### Description of the invention

The invention achieves the object by the features of the claims and especially provides retroviral particles that are alpharetrovirus-based particles, which comprise or consist of a protein component and at least one RNA construct in association, especially packaged into particles, via the protein component and a corresponding binding RNA hairpin structure. The alpharetrovirus-based protein component particles comprises or consists of the domains which are an alpharetroviral matrix protein (MA), an alpharetroviral p2 protein (p2), an alpharetroviral p10 protein (p10), an alpharetroviral capsid protein (CA), an alpharetroviral nucleocapsid protein (NC) and optionally an additional alpharetroviral protease protein (PR), a linker which may be a viral protease site or which may be devoid of a protease site, and an MS2 bacteriophage-derived MS2 coat protein dimer (2xMS2CP), preferably in this order from N-terminus to C-terminus, and preferably a pseudotyping protein, wherein the pseudotyping protein may optionally include a targeting protein, ligand or peptide. This pseudotyping protein during production of the alpharetrovirus-based particles is e.g. expressed in cultivated producer cells from a separate expression cassette. The protein component between the domains contains protease sites such that during production of alpharetrovirus-based particles, one protein comprising or consisting of the alpharetroviral domains of MA-p2-p10-CA-NC or MA-p2-p10-CA-NC-PR, each followed by the 2xMS2CP, can be proteolyzed by a viral protease, e.g. to generate separate proteins containing only one of the alpharetroviral domains of MA, p2, p10, CA, or NC, or at least two connected alpharetroviral domains. The protease sites that are arranged between each of the alpharetroviral domains can be the natural protease sites of the alpharetrovirus. In the absence of protease activity in producer cells, the Gag-MS2 protein component can be present in the alpharetrovirus-based particles as one single-chain protein. It has been found that fully functional, e.g. transduction-competent, alpharetrovirus-based particles, according to the invention, can be produced that contain a protein component that contains protease sites between its alpharetroviral domains, whose protein component is present as one chain, e.g. comprising MA-p2-p10 -CA-NC-2xMSCP. In the alternative, the protein component can be devoid of protease sites between its domains, e.g. the alpharetroviral domains of the protein component can be connected by linkers that are no protease sites.

It was found that during production of functional alpharetrovirus-based particles in producer cells, a viral protease site arranged between the C-terminal NC domain of the alpharetroviral protein component and the MS2 coat protein dimer is not necessarily hydrolysed. Therefore, optionally, the alpharetroviral domains of MA-p2-p10-CA-NC or MA-p2-p10-CA-NC-PR can be linked to the MS2 coat protein, e.g. 2xMS2CP, by a linker that contains no protease site, especially no viral protease site, e.g. a linker having a length of 6 to 25 amino acids, preferably of up to 20 amino acids. Herein, the alpharetroviral domains of MA-p2-p10-CA-NC or MA-p2-plO-CA-NC-PR, optionally including protease sites between each of the domains, are also referred to as a.Gag.

The pseudotyping protein can be expressed from an expression cassette that is separate from the expression cassette encoding the alpharetroviral protein domains. The pseudotyping protein can e.g. be selected from VSVg, other strains of the VSV family, RD114, BaeV, MARAV, COCV, ecotropic or amphotropic MLV Env, GALV, Measles envelope, NipahV envelope. Blinded Measles and NipahV envelopes may be fused to a targeting protein, ligand, scFv or receptor binding domain. It was found that the target cell specificity of the alpharetrovirus-based particle of the invention can be determined by its pseudotyping protein.

The alpharetrovirus-based particles have the advantage of containing in association with the protein component at least one RNA construct, which may be a non-coding RNA, e.g. shRNA, sgRNA, IncRNA, miRNA, shRNA/miRNA hybrid, or which may be an RNA encoding at least one transgene, also called a gene of interest (GOI), which can be a coding sequence for a protein, e.g. encoding an antigen for eliciting an immune reaction in a recipient, a transcription factor, or a coding sequence encoding a gene editing nuclease, e.g. Cas9, preferably the latter in combination with at least one additional RNA construct, e.g. an sgRNA, optionally in combination with at least two or three additional different sgRNAs. Generally, each sgRNA (single-guide RNA) is provided for co-operating with Cas9 nuclease.

For the RNA construct encoding a protein, the RNA construct preferably from 5' to 3' contains the following elements: Cap - GOI - 2xTS - optionally PRE - polyA tail, wherein Cap is a cap structure consisting of an N7-methylated GTP molecule linked to the first transcribed nucleotide serving as a ribosomal initiation site, GOI is the coding sequence, 2xTS is two adjacent MS2 target sites.

Alpharetrovirus-based particles that encode a protein which is an antigen, are a vaccine, e.g. for use in eliciting an immune response and/or for generating an immune protection against an infectious agent, which is e.g. a virus or a bacterium, and/or for treatment of autoimmune and/or cancer diseases. Exemplary antigens are proteins of viral origin or of bacterial origin or cancer antigen for use of the alpharetrovirus-based particles encoding an antigen in the generation of an immune protection, e.g. spike protein of a coronavirus, e.g. of SARS-CoV2, GP120 of HIV-1, Ag85 (e.g. according to https://pubmed.ncbi.nlm.nih.gov/23035231/) of Mycobacterium tuberculosis, OVA (ovalbumin), or for cancer therapy, CEA as an embryonic cancer antigen, tumor neoantigens (e.g. NY-ESO-1, MAGE-A3) or asparaginyl endopeptidase legumain.

Generally herein, RNA constructs can also be referred to as transcripts, because the RNA constructs that are contained in the alpharetrovirus-based particles are produced as transcripts from a coding or non-coding DNA sequence.

The RNA constructs contained in the alpharetrovirus-based particles are bound and incorporated via the 2xMS2CP of the protein component that interacts with the corresponding MS2 target site (TS) which is present on the RNA construct. The TS comprises or consists of two genetically fused hairpin structures that are present in each RNA construct. In RNA constructs that encode a protein, the TS preferably is placed adjacent to the gene of interest encoding section and in 5' of a PRE element with a downstream adjacently arranged poly A tail.

In RNA constructs, which e.g. are sgRNA, shRNA, miRNA, shRNA/miRNA hybrids and/or IncRNA, the TS domains are either integrated within the sgRNA, shRNA, miRNA, and/or IncRNA section or are placed downstream adjacent, e.g. in 3', to the sgRNA, shRNA, miRNA, and/or IncRNA section. Similarly, for other non-coding RNA constructs, the TS domains can be incorporated or arranged adjacently. In addition, e.g. the non-coding RNA, e.g. sgRNA, can be arranged between the hairpin sections of two TS domains, e.g. the sgRNA, shRNA, miRNA, IncRNA or a combination of at least two of these, may link two TS hairpin sections, especially may link two stem sections of two TS hairpin sections.

Generally, the alpharetrovirus-based particles of the invention may have a lipid coat that arises from their derivation of a producer cell, e.g. due to the budding the alpharetrovirus-based particles may comprise portions of the plasma membrane of the producer cell. The alpharetrovirus-based particle comprises or consists of an a.Gag MS2 protein component comprising or consisting of the domains of alpharetroviral MA-p2-p10-CA-NC-2xMS2CP or MA-p2-p10-CA-NC-PR-2xMS2CP, in each case optionally with a protease site between each of the domains, the a.Gag protein component preferably having at least 90%, preferably at least 95% identity to an amino acid sequence encoded by nucleotides No. 1462 to 3207 of SEQ ID NO: 1, e.g. an amino acid sequence encoded by nucleotides No. 1462 to 3186 of SEQ ID NO: 1. In the preferred a.Gag protein component, the domains are separated by viral protease sites, and these domains are separated e.g. by a first linker which may be or contain a viral protease site having at least 90%, preferably at least 95% identity to an amino acid sequence encoded by nucleotides 3187 to 3207 of SEQ ID NO: 1, optionally an additional second linker between the first linker and the MS2 coat protein (2xMS2CP), the second linker e.g. having at least 90%, preferably at least 95% identity to an amino acid sequence encoded by nucleotides No. 3208 to 3222 of SEQ ID NO: 1, and the MS2 coat protein dimer (2xMS2CP), preferably having at least 90%, preferably at least 95% identity to an amino acid sequence encoded by nucleotides No. 3223 to 3999 of SEQ ID NO: 1, and a pseudotyping protein. In association with the protein component, at least one RNA construct, which is a transgene encoding and/or non-coding RNA transcript, is linked to at least one target site (TS), preferably at least two target sites (2x TS). The at least one TS, preferably at least two TS, of each RNA construct during production in producer cells associates with the MS2 coat protein dimer (2xMS2CP), resulting in the RNA constructs being associated, or packaged, in the protein component. The pseudotyping protein VSVg may have an amino acid sequence encoded by nucleotides 1241 to 2776 of SEQ ID NO: 4

Each of the MS2 target sites (TS) can have a nucleotide sequence of at least 90%, preferably at least 95% to at least one sequence selected from nucleotides 1943 to 1965 and/or nucleotides 1982 to 2004 of SEQ ID NO: 2, of nucleotides 1681 to 1701 and/or nucleotides 1751 to 1771 of SEQ ID NO: 3, of nucleotides 1755 to 1775 and/or nucleotides 1794 to 1814 of SEQ ID NO: 14, of nucleotides 5697 to 5719 and/or nucleotides 5736 to 5758 of SEQ ID NO: 5, of nucleotides 2337 to 2359 and/or nucleotides 2376 to 2398 of SEQ ID NO: 6. Therein, each of at least two target sites can have the same or different nucleotide sequences. Preferably, the target sites are spaced by 60 to 40 nucleotides (nt), e.g. spaced by 50 nt, which spacing nucleotides can comprise a non-coding RNA, e.g. sgRNA, shRNA, miRNA. Two target sites (2x TS) can e.g. have a nucleotide sequence of nucleotides 1912 to 2045 of SEQ ID NO: 2, and/or of nucleotides 1982 to 2004 of SEQ ID NO: 2, or of nucleotides 1681 to 1701 and, spaced by 60 to 40 nt, e.g. by 50 nt, nucleotides 1751 to 1771 of SEQ ID NO: 3, e.g. nucleotides 1665 to 1800 of SEQ ID NO: 3, or of nucleotides 5666 to 5799 of SEQ ID NO: 5, or of nucleotides 2306 to 2439 of SEQ ID NO: 6.

The alpharetrovirus-based particle according to the invention is suitable for use for introducing at least one RNA construct into a target cell, e.g. for expressing the gene of interest from its mRNA that is contained in the alpharetrovirus-based particle, and/or for introducing RNA molecules, e.g. at least one, preferably at least two non-coding RNAs, e.g. selected from sgRNA, siRNA, shRNA, IncRNA, tRNA, rRNA,_ or a combination of at least two of these, and/or RNA encoding a protein. In addition, self-amplifying RNA replicons encoding any of the above RNA species may be transferred.

In the process for producing the alpharetrovirus-based particle according to the invention, the a.Gag.MS2 protein component comprising alpharetroviral MA-p2-p10-CA-NC or MA-p2-p10-CA-NC-PR, a linker which may contain a viral protease site (pr), the MS2 coat protein dimer (2xMS2CP) having at least 90%, preferably at least 95% identity to the amino acids encoded by nucleotides 1462 to 3208 of SEQ ID NO: 1, wherein the MA-p2-plO-CA-NC-PR, a linker which may contain a viral protease site (pr), the MS2 coat protein dimer (2xMS2CP) having at least 90%, preferably at least 95% identity to the amino acids encoded by nucleotides No. 1462 to 3594 of SEQ ID NO: 12, and a pseudotyping protein and at least one RNA construct encoding a gene and/or a coding or non-coding RNA species of interest linked to at least one target site (TS), preferably linked to at least two target sites (2x TS), are concurrently expressed in a producer cell, e.g. from expression cassettes under the control of a polymerase II (Pol II) promoter element or under the control of a polymerase III (Pol III) promoter element, e.g. the CMV promoter, PGK promoter, EF1a promoter, or the Beta-actin promoter as examples for Pol II promoters , as well as the hU6 and H1 as examples for Pol III promoters.

Expression cassettes that encode the respective a.Gag.MS2 protein component or encode an RNA construct containing a GOI or protein encoding sequence, preferably at their 3'-end encode a poly adenylation signal (pA), optionally a PRE adjacent to the 5'-end of the poly adenylation signal. The at least one RNA construct containing a GOI or protein encoding sequence, whose RNA is contained in an alpharetrovirus-based particle and at its 5'-end has a Cap structure and at its 3'-end has a poly adenylation tail.

Surprisingly, it was found that the a.Gag.MS2 protein component effectively packages RNA constructs, e.g. at least one, two, or three different RNA constructs within one alpharetrovirus-based particle, the protein component of which comprises or consists of the alpharetroviral domains MA,p2, p10, CA, NC, optionally PR, and the MS2 coat protein dimer (2xMS2CP), and a pseudotyping protein, e.g. VSVg. Unexpectedly, the 2xMS2CP portion of the protein component together with the alpharetroviral MA, p2, p10, CA, NC, and optionally PR, protein domains and the pseudotyping protein forms alpharetrovirus-based particles that contain at least one RNA construct in producer cells. Further, it was unexpected that these particles effectively associate with human or murine target cells and efficiently transduce the target cells for transporting the RNA constructs into the target cells. Generally herein, target cells are any cells, to which the alpharetrovirus-based particle of the invention bind, whose binding is e.g. determined by the choice of the pseudotyping protein of the alpharetrovirus-based particle.

Generally, nucleic acid sequence elements are given in their arrangement from 5' to 3', unless indicated otherwise, and amino acid sequences are given in their arrangement from N-terminus to C-terminus.

The invention is now described in greater detail and with reference to the Figures, which show in
- Fig. 1A a schematic of nucleic acid constructs for a comparative gammaretrovirus-based g.Gag.MS2 particle production,
- Fig. 1B a schematic of nucleic acid constructs for a.Gag.MS2 alpharetrovirus-based particle generation according to the invention,
- Fig. 1C a schematic of the FLP recombinase indicator gene cassette and results of recombination of a reporter gene cassette in murine SC1 cells after transduction with comparative analysis of three types of alpharetrovirus-based particles transferring FLP recombinase (a.NC.MS2, a.NC.pr.MS2, a.PR.pr.MS2) according to the invention, boxed is the best working variant (a.NC.pr.MS2), which is also used in the following figures,
- Fig. 1D results of FLP-mediated recombination in reporter cells from Fig. 1C with a comparative gammaretrovirus-based particle (g.Gag.MS2) and with the best working alpharetrovirus-based particle (a.NC.pr.MS2) according to the invention,
- Fig. 1E results of FLP-mediated excision of a reprogramming gene (OKSM) cassette in human iPSC after transduction with a comparative gammaretrovirus-based particle (g.Gag.MS2) and with an alpharetrovirus-based particle (a.NC.pr.MS2) according to the invention,
- Fig. 1F results of expression of firefly luciferase in human HT1080 cells after transduction with a comparative gammaretrovirus-based particle (g.Gag.MS2) and with an alpharetrovirus-based particle (a.NC.pr.MS2) according to the invention,
- Fig. 1G results of expression of firefly luciferase in primary human newborn foreskin fibroblasts (NuFF) after transduction with a comparative gammaretrovirus-based particle (g.Gag.MS2) and with an alpharetrovirus-based particle (a.NC.pr.MS2) according to the invention,
- Fig. 2A copy numbers of mRNA in supernatants of alpharetrovirus-based particles (a.NC.pr.MS2) according to the invention and in comparative gammaretrovirus-based particles (g.Gag.MS2),
- Fig. 2B titers (particle / µL) of supernatants with alpharetrovirus-based particles (a.NC.pr.MS2) according to the invention and of comparative gammaretrovirus-based supernatants (g.Gag.MS2) as well as non-retroviral extracellular vesicle controls (pcDNA3 only and VSVg only),
- Fig. 2C results of particle size measurements of alpharetrovirus-based particles (a.NC.pr.MS2) according to the invention and of comparative gammaretrovirus-based particles (g.Gag.MS2) as well as non-retroviral extracellular vesicle controls (pcDNA3 only and VSVg only),
- Fig. 2D transmission electron micrographs of virus-like particles of the invention and of comparative virus-like particles, incl. control vector particles that were packaged with respective wildtype (wt) gammaretroviral or alpharetroviral Gag-Pol (wt.g.Gag-Pol and wt.a.Gag-Pol);
- Fig. 2E results of the determination of firefly luciferase transgene expression in target cells transduced with the alpharetrovirus-like particles (a.NC.pr.MS2) and with comparative gammaretrovirus-like particles (g.Gag.MS2) and respective integrating controls,
- Fig. 3A schematically shows the components used for production of comparative gammaretrovirus-based particles (g.Gag.MS2) that deliver CRISPR/Cas9 components into target cells,
- Fig. 3B schematically shows the components used for production of CRISPR/Cas9 RNA-delivering alpharetrovirus-based particles (a.NC.pr.MS2) according to the invention,
- Fig. 3C RFP657.Tet2 reporter knockout rates with a.NC.pr.MS2-based CRISPR/Cas9 particles that were packaged with mouse Tet2-targeting sgRNAs carrying the MS2 target site either within (TS.inc) or adjacent to (TS.adj) the sgRNA scaffold,
- Fig. 3D copy numbers of CRISPR/Cas9 RNA (SpCas9.TS mRNA and Tet2.TS sgRNA transcripts) in supernatants of alpharetrovirus-based particles (a.NC.pr.MS2; here Tet2.TS.adj) according to the invention and in comparative gammaretrovirus-based (g.Gag.MS2; here Tet2.TS.inc) supernatants,
- Fig. 3E knockout rates of a RFP657.Tet 2 reporter gene construct in human HT1080 target cells,
- Fig. 3F knockout rates of Tet2 target gene reporter construct in human iPS cells,
- Fig. 4 knockout of endogenous CXCR4 gene in human Jurkat cells by gammaretrovirus-based (g.Gag.MS2) and alpharetrovirus-based (a.NC.pr.MS2) CRISPR/Cas9 all-in-one particles. In (A) representative FACS results are shown, in (B) experiments in biological replicates in comparison to integrating lentiviral LIT.CXCR4 CRISPR/Cas9 all-in-one particles and non-targeting mouse Trp53 control Gag.MS2 particles,
- Fig. 5A to 5D alpharetrovirus-based (a.NC.pr.MS2) particle-mediated knockout rates of the endogenous human TP53 gene in primary human NuFF cells (A), in primary human hepatocytes (PHH) (B), in human CD34+ hematopoietic stem and progenitor cells (HSPC) (C), and in primary murine embryonic fibroblasts (MEF) (D) by application of different MOIs (multiplicity of infection), expressed as applied SpCas9.TS mRNA copies per cell,
- Fig. 6A and 6B multiplexing experiments with use of CRISPR/Cas9-delivering alpharetrovirus-based particles containing two sgRNAs (Fig. 6A, knockout of CXCR4 and RFP657.Tet2) or three sgRNAs (Fig. 6B, in addition to A also EGFP was knocked out), and
- Fig. 7A exemplary expression plasmids for alpharetrovirus-based particles containing VEE self-amplifying RNA replicons (with non-structural proteins nsP1, nsP2, NsP3, nsP4) and EGFP as gene of interest, and in Fig. 7B results from transduction of human HT1080 target cells with alpharetrovirus-based particles of Fig. 7A.

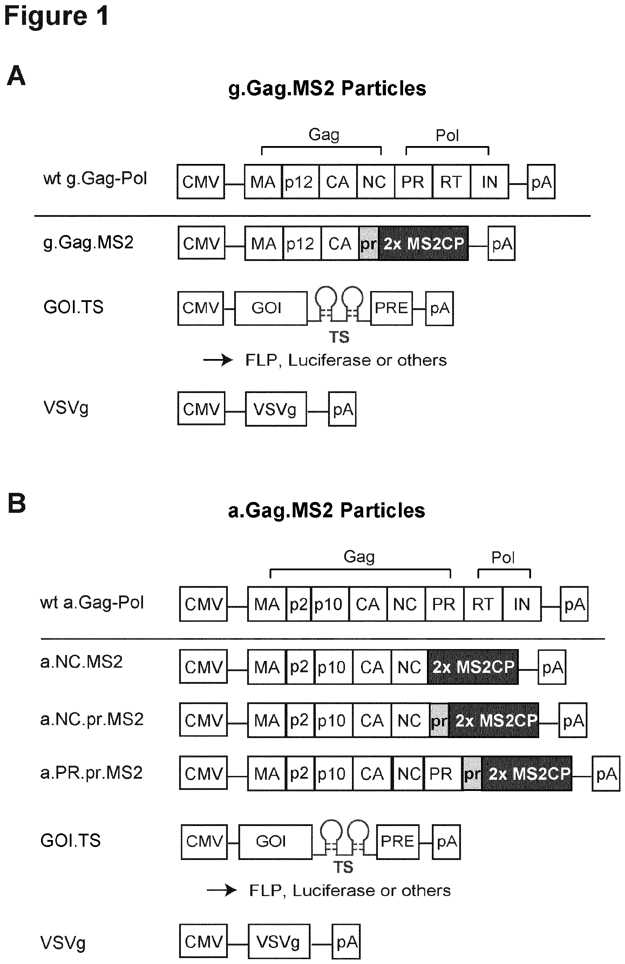

The nucleic acid constructs for producing in a packaging cell the mRNA-delivering alpharetrovirus-based particle (a.Gag.MS2 Particles) according to the invention are schematically depicted in Fig. 1B, and Fig. 1A schematically depicts comparative nucleic acid constructs for producing gammaretrovirus-based particles (g.Gag.MS2 Particles). In addition, Fig. 1A and Fig. 1B schematically depict a nucleic acid construct for expressing wild-type alpharetroviral particles (wt a.Gag-Pol) or gammaretroviral particles (wt g.Gag-Pol).

Fig. 1A shows the expression constructs for packaging of mRNA transferring gammaretroviral Gag.MS2 (g.Gag.MS2) particles. 293T producer cells were transfected with expression plasmids encoding for g.Gag.MS2, the envelope glycoprotein (exemplified by VSVg), and the gene of interest (GOI)-carrying mRNA. The latter also encodes two copies of the MS2 hairpin structure (TS), which allows specific packaging of transcribed GOI.TS mRNA into arising particles by the genetically fused MS2 coat protein dimer (2x MS2CP) within g.Gag.MS2. The MS2CP dimer protein is separated from the gammaretroviral nucleocapsid (NC) protein by the natural viral protease sites (pr, light gray box) which here functions as a linker, as the gammaretroviral Pol enzymes including the viral protease (PR) are not added during g.Gag.MS2 particle production. The configuration of gammaretroviral wildtype (wt) Gag-Pol is shown on top. CMV: Cytomegalovirus promotor; PRE: Woodchuck Hepatitis Virus post-transcriptional regulatory element; pA: PolyA signal. MA: viral matrix protein, p12: viral p12 protein, CA: viral capsid protein, RT: viral reverse transcriptase, IN: viral integrase.

Fig. 1B shows the expression constructs for production of alpharetroviral Gag.MS2 (a.Gag.MS2) particles. Packaging of GOI mRNA-delivering a.Gag.MS2 particles is shown for three different alpharetroviral a.Gag.MS2 variants, a.NC.MS2, a.NC.pr.MS2 and a.PR.pr.MS2. In the a.NC.MS2 variant, the MS2CP dimer is directly fused to the alpharetroviral NC domain, whereas in a.NC.pr.MS2 NC and the MS2CP dimer were separated by a linker, which in this case is the natural viral protease site (pr) (incl. extended linker) indicated by a light gray box. Particles packaged with a.PR.pr.MS2 in addition contain viral PR to promote particle maturation after budding. In this variant, the MS2CP dimer is separated by PR at the natural viral protease site (pr; light gray box) at this position.

The alpharetrovirus-based a.Gag.MS2 protein component in alpharetrovirus-based particles of the invention, which is expressed as a translation product, as schematically depicted in Fig. 1B, comprises or consists of MA-p2-p10-CA-NC portion, wherein the domains are linked by protease recognition sites, and the 2xMS2CP portion, which are produced as one fusion protein (a.NC.pr.MS2), which includes as a linker an extended viral protease site (pr) between the N-terminal MA-p2-p10-CA-NC and the C-terminal 2xMS2CP from one common coding sequence. The domains of the MA-p2-p10-CA-NC portion in the presence of a viral protease are separable at protease sites arranged between the domains. In this embodiment, the alpharetroviral portion does not contain the PR domain of the wild-type Gag protein but is expressed as a translation product that consists of MA-p2-p10-CA-NC in a fusion protein with the protease recognition site pr and the 2xMS2CP portion. Further, the alpharetroviral portion, and respectively the alpharetrovirus-based particle, does not contain viral enzymatic proteins, e.g. no reverse transcriptase nor integrase and/or no viral protease. The one common coding sequence for a fusion protein is depicted in Fig. 1B as a.Gag.MS2, containing as a DNA the coding sequence for MA-p2-p10-CA-NC-viral protease site-2xMS2CP of nucleotides 1462 to 3207 of SEQ ID NO: 1 including in an extended linker containing the protease site and Glycins, which separates NC from MS2, and the MS2 coat protein dimer encoded by nucleotides 3223 to 3999 of SEQ ID NO: 1 under the control of a promoter, exemplified by a CMV promoter of nucleotides 232 to 819 of SEQ ID NO: 1, with a 3'-terminal poly A signal (pA) of nucleotides 4078 to 4302 of SEQ ID NO: 1.

As a further embodiment, Fig. 1B shows the arrangement of the coding sequence for MA-p2-p10-CA-NC-PR-viral protease site-2xMS2CP (a.PR.pr.MS2), which contains the PR domain arranged between the NC domain and the extended viral protease site linker. The PR domain encodes the alpharetroviral protease. The one common coding sequence for MA-p2-p10-CA-NC-PR-viral protease site-2xMS2CP is contained in SEQ ID NO: 12, wherein in a codon-optimized sequence nucleotides 1462 to 3594 encode the polyprotein MA-p2-p10-CA-NC-PR-viral protease site, nucleotides 3595 to 3612 encode an adjacent linker, and nucleotides 3613 to 4389 encode the adjacent MS2 coat protein dimer, wherein these coding sequences encode one fusion protein. The plasmid backbone of SEQ ID NO: 12 is from pcDNA3.

Fig. 1B depicts the arrangement of a coding sequence for MA-p2-p10-CA-NC-2xMS2CP (a.NC.MS2), which does not contain the extended viral protease recognition site linker (pr) between the NC domain and the 2xMS2CP.

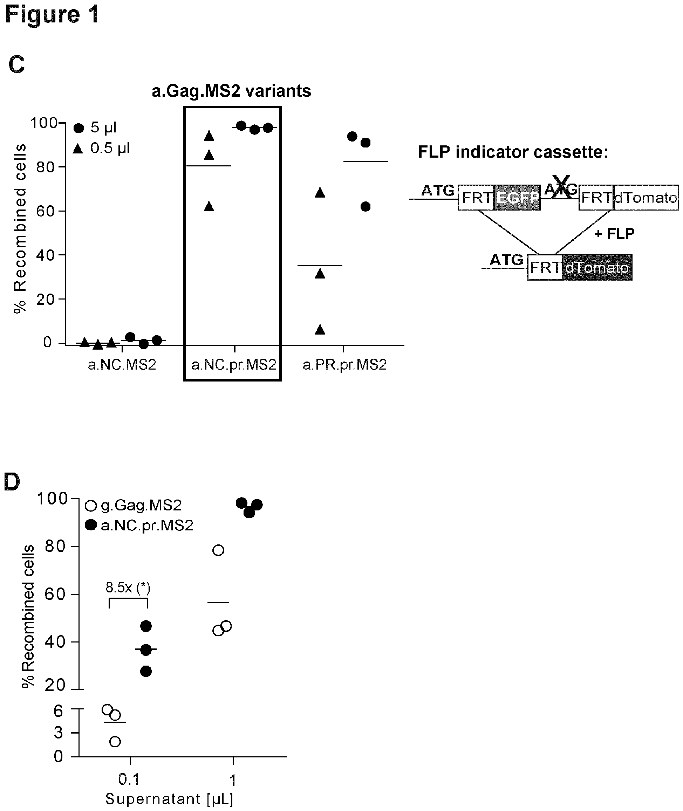

When producing the alpharetroviral particles in producer cells, the DNA is transcribed into RNA, e.g. an mRNA with the coding sequence. The poly A signal effects the addition of the polyadenylation tail to the mRNA, and this mRNA is translated into the protein component. The viral protease sites arranged between the domains of MA-p2-p10-CA-NC in the presence of the viral protease allow the proteolysis into the single domains, and assembly of enveloped/pseudotyped alpharetrovirus-based particles which comprise the MS2 coat protein in association with RNA molecules that contain at least one, preferably two or more, target sites (TS). When producing the alpharetrovirus-based particles in the absence of proteolytic activity for the protease sites between the alpharetroviral domains, e.g. MA-p2-p10-CA-NC-viral protease site-2xMS2CP from SEQ ID NO: 1, it was found that this protein component was present as one amino acid chain, with essentially no proteolytic processing observed in a Western blot. Accordingly, in the protein component of the invention comprising or consisting MA-p2-p10-CA-NC-viral protease site-2xMS2CP, protease sites between the domains can be non-functional, e.g. the domains can be linked by linkers that contain no protease sites, and accordingly the gag polyprotein component can be present as one single protein. This shows that the protease sites between the alpharetroviral domains of the gag polyprotein component need not be proteolytically cleaved when producing functional alpharetrovirus-based Gag.MS2 particles of the invention. Noteworthy, the presence of an extended linker harboring 3' to NC an extended protease site linker (composed of a protease site and glycin linker) 5' to 2xMS2CP was important for functionality (see Fig. 1C, shown by comparison of a.NC.MS2 and a.NC.pr.MS2).

In the embodiment of the protein component comprising a protease PR, e.g. MA-p2-p10-CA-NC-PR-viral protease site-2xMS2CP (a.PR.pr.MS2) encoded by SEQ ID NO: 12, it was found that the presence of the viral protease also resulted in production of functional alpharetrovirus-based particles of the invention (a.PR.pr.MS2 in Fig. 1B and C). Preferably, the 2xMS2CP can be cleaved off from the alpharetroviral Gag (MA-p2-p10-CA-PR) part.

The effective transduction of target cells and activity of the transferred RNAs show that from the a.NC.pr.MS2 and a.PR.pr.MS2 variants functional a.Gag.MS2 particles are effectively formed. The fact that both variants are capable of transferring functional RNAs illustrates that the protease and possible proteolytic cleavage into the Gag.MS2 subdomains is not essential for functionality. Accordingly, in these a.Gag.MS2 particles, the viral protease sites between the viral Gag subdomains and between Gag and 2xMS2CP can be non-hydrolysed (e.g. a.NC.pr.MS2) or optionally can be hydrolysed (e.g. a.PR.pr.MS2) to generate separate or linked domains of MA, p2, p10, CA, NC, PR and, preferably 2xMS2CP can be cleaved off from the gag part.

In producer cells, the RNA constructs are transcribed from a DNA under the control of a promoter, exemplified by the CMV promoter, and comprise or consist of the coding sequence of the gene of interest (GOI), at least one, preferably at least two target sites, and poly A signal, optionally with a PRE between the GOI and the poly A signal (GOI.TS). The resulting RNA from 5' to 3' consists of the coding sequence of the GOI, the at least one TS, preferably at least two TS, optionally a PRE, and preferably a poly adenylation tail. In the examples, two target sites (TS) are shown, which interact with the MS2CP protein for associating, incl. packaging, the RNA construct into forming a.Gag.MS2 particles. A GOI can e.g. be a gene editing nuclease, transposase, recombinase, enzyme, or other cellular proteins, a non-coding RNA, e.g. sgRNA, an shRNA (small hairpin RNA), miRNA, IncRNA, or a combination of coding and non-coding RNAs. Preferably, the RNA containing non-coding constructs are transcribed from a DNA under the control of a polymerase III promoter, e.g. the human U6 (hU6) or H1 promoters.

According to the invention, the alpharetrovirus-based particles may contain at least one, two, three or more RNA coding and non-coding constructs, each RNA construct with a TS incorporated into the molecule (e.g. incorporated or adjacent), optionally a PRE (posttranscriptional regulatory element, e.g. the PRE of the woodchuck hepatitis virus), and, especially for protein-encoding RNA and miRNAs, more preferably a 3'-terminal poly adenylation tail.

Generally preferred, a coding GOI and optionally for non-coding GOI, e.g. IncRNA, miRNA and shRNA/miRNA hybrid, can be transcribed from a DNA under the control of a Pol. II promoter, e.g. the CMV promoter, preferably the GOI followed in 3' with at least one adjacent or incorporated TS, polyA signal, optionally a PRE. A non-coding GOI, especially shRNA, sgRNA and other short non-coding RNA, can be transcribed from a DNA under the control of a Pol. III promoter, e.g. hU6, H1 promoter, the non-coding RNA preferably followed in 3' with a Pol. III termination signal (polyT stretch, TTTTT).

For a.Gag.MS2 particle generation, the addition of a pseudotype envelope protein is necessary. Thus, the producer cell contains a transfected or stably expressed construct for expressing a pseudotyping protein, e.g. VSVg (glycoprotein of the vesicular stomatitis virus), under the control of a promoter, exemplified by a CMV promoter, optionally an intron, and a 3'-terminal poly adenylation signal (CMV-VSVg-pA). The pseudotyping protein, when expressed in a producer cell, is incorporated into the lipid envelope of the alpharetrovirus-based particle.

Preferably, the VSVg cDNA can be substituted by another envelope protein that is suitable for pseudotyping retroviral, incl. gammaretroviral, alpharetroviral and lentiviral, vectors and the here described a.Gag.MS2 particles can be used (see list of envelope proteins provided above).

### Example 1: Packaging and performance of alpharetrovirus-based particles delivering FLP recombinase or firefly luciferase mRNAs

The components used for production of alpharetrovirus-based Gag.MS2 (a.Gag.MS2) particles are schematically shown in Fig. 1B. 293T producer cells were transfected with expression plasmids encoding for a.Gag.MS2 (SEQ ID NO: 1), the VSVg envelope glycoprotein is expressed from SEQ ID NO: 4, and the gene of interest (GOI)-carrying mRNA is transcribed from SEQ ID NO: 2 (FLP) and SEQ ID NO: 6 (firefly luciferase). The mRNA encoding the FLP GOI also encodes two copies of the MS2 hairpin structure (TS) at nucleotides 1943 to 1965 and at nucleotides 1982 to 2004 of SEQ ID NO: 2, whose TS allow specific packaging of transcribed GOI.TS mRNA into MS2 coat protein dimer (2x MS2CP) of arising alpharetrovirus-based particles. Correspondingly, the mRNA encoding the firefly luciferase also encodes two copies of the MS2 hairpin structure (TS) at nucleotides 2337 to 2359 and at nucleotides 2376 to 2398 of SEQ ID NO: 6.

For comparison, a gammaretroviral protein component g.Gag.MS2 correlate is used for producing comparative gammaretrovirus-like particles, with mRNA encoding the same GOIs. As shown in Fig. 1A, the comparative gammaretrovirus-based particles g.Gag.MS2 also contained an extended protease recognition site pr (light gray box) between the retroviral domains and the 2xMSCP, and were also pseudotyped by VSVg.

CMV: Cytomegalovirus promoter; PRE: Woodchuck Hepatitis Virus posttranscriptional regulatory element; pA: PolyA signal.

For the production of alpharetrovirus-based Gag.MS2 particles, viral supernatants were produced after transient transfection of 293T producer cells using standard calcium phosphate DNA precipitation. Briefly, the day before transfection, 5 to 6 x 10⁶cells were seeded per 10 cm diameter culture dish (Sarstedt, Nümbrecht, Germany). Viral particles were generated by co-transfection of human embryonic kidney 293T (293T) cells with plasmids encoding a.Gag.MS2 (SEQ ID NO: 1), GOI.TS (SEQ ID NO: 2 for FLP and SEQ ID NO:6 for firefly luciferase) containing the coding sequence for FLP recombinase with TS or firefly luciferase with TS, as well as PRE and poly adenylation signal, and an expression plasmid containing the coding sequence for VSVg (SEQ ID NO: 4).

The alpharetrovirus-based Gag.MS2 particles were concentrated 100-fold by ultracentrifugation at 82,740 x g for 2 h or at 13,238 x g overnight at 4°C, and used as alpharetrovirus-based particle supernatant.

For comparison, gammaretroviral particles were produced using the same protocol but using the coding sequence for the gammaretroviral Gag.MS2 expression construct. of SEQ ID NO: 13.

Respective alpharetroviral Gag.MS2 particle functionality and activity of the RNA contained in particles were assessed with the help of a cell-based FLP recombinase (FLP) reporter system (Fig. 1C). For this purpose, different a.Gag.MS2 particles delivering FLP recombinase (FLP) mRNA as the GOI.TS were produced as depicted in Figure 1B and tested on SC1-based FLP reporter cells, in which FLP-mediated recombination is indicated by the switch from EGFP to dTomato expression.

FLP reporter cells contain a reporter construct (FLP indicator cassette), in which EGFP (enhanced green fluorescent protein) is flanked by two FRT (FLP recognition target) sites, and in 3' to this contained the red-fluorescent dTomato as a second reporter gene, which lacks the ATG start codon. Thus, the activity of FLP results in the excision of EGFP and induces dTomato expression. As the cells contained the FLP indicator cassette in three copies, incomplete recombination of all three alleles by FLP also results in cells that express both dTomato and EGFP. Those and dTomato only expressing cells were both considered as recombined cells as indicated on the Y-axis of the graph.

Variants a.NC.pr.MS2 and a.PR.pr.MS2 outperformed variant a.NC.MS2. The best performing variant a.NC.pr.MS2 is boxed and is used in the following analyses. This shows that, preferably, within the a.Gag.MS2 protein component the 2xMS2CP should be separated from Gag subdomains by a linker region.

Fig. 1D shows the highly efficient transfer of FLP mRNA by the best performing alpharetrovirus-based Gag.MS2 particles (a.NC.pr.MS2; (•)) in comparison to their gammaretrovirus-based correlate (g.Gag.MS2, Fig. 1A, (∘)). SC-1-based FLP reporter cells containing the FLP indicator cassette were transduced with indicated volumes of g.Gag.MS2.FLP or a.NC.pr.MS2.FLP supernatants. The Y-axis shows the percentage of recombined cells, which emerged from FLP-mediated excision of an FRT-flanked EGFP. The alpharetrovirus-based a.NC.pr.MS2.FLP particles show enhanced FLP-mediated recombination.

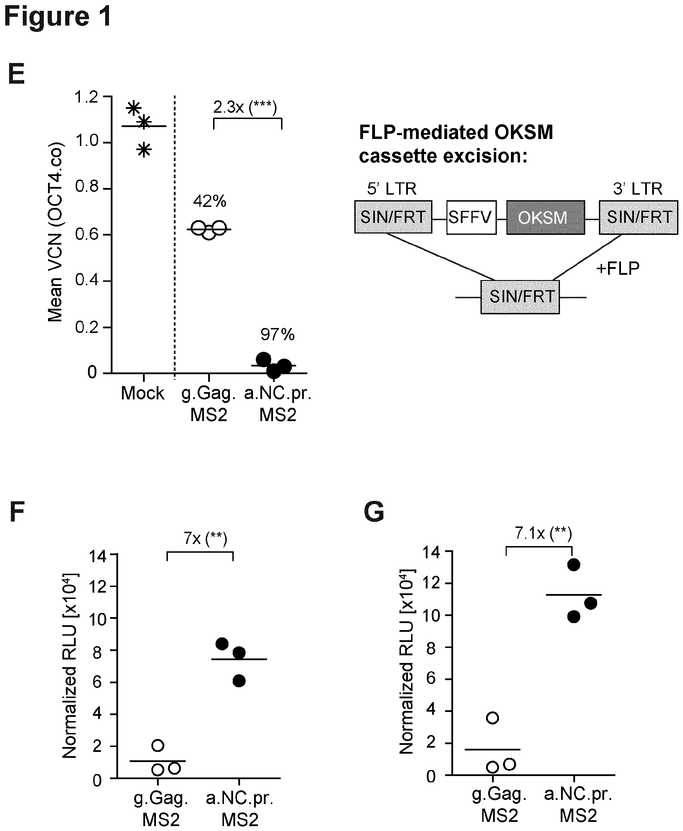

Fig. 1E shows the result of highly efficient removal of the reprogramming vector cassette (SFFV.OKSM) from the genome of human induced pluripotent stem cells (iPSC) via exemplary a.Gag.MS2 particles (a.NC.pr.MS2) containing the FLP recombinase as gene of interest. Human iPSC cells harboring one SFFV.OKSM vector integration containing the reporter cassette were treated with comparative gammaretrovirus-based (∘) g.Gag.MS2 particle supernatants or alpharetrovirus-based (•) a.Gag.MS2 particle supernatants.

Excision of SFFV.OKSM from the reporter cassette, schematically shown in Fig. 1E (FLP-mediated OKSM cassette excision) was analyzed by quantitative real-time PCR using primers detecting the codon-optimized (co) OCT4 gene (O), which is part of the OKSM reprogramming cassette (VCN: Vector copy number; LTR: Long terminal repeats; SIN/FRT: Self-inactivating LTR with FRT site). The results show that the delivery of FLP mRNA is much more efficient (97%) by alpharetrovirus-based particles compared to gammaretrovirus-based particles (42%).

As a further GOI, firefly luciferase mRNA was packaged (GOI.TS, nucleotides 624 to 2439 of SEQ ID NO: 6) in alpharetrovirus-based a.Gag.MS2 particles and in comparative gammaretrovirus-based g.Gag.MS2 particles as shown in Fig. 1A and 1B. The results show superior transfer of firefly luciferase mRNA by the alpharetrovirus-based a.Gag.MS2 particles. The graphs depict relative light units (RLU) normalized to total protein in human HT1080 cells (Fig. 1F) or primary human newborn foreskin fibroblasts (NuFF) (Fig. 1G).

In case of Gag.MS2 "mRNA only" delivery, the copy numbers of the encoding mRNA in 100-fold concentrated supernatants were determined by quantitative real-time RT (reverse transcription) PCR (qRT-PCR). To eliminate potential plasmid contamination, supernatants were treated with 2 units TURBO DNase (Ambion/ Thermo Fisher Scientific) for 1 h at 37°C. Subsequently, the RNA was extracted from Gag.MS2 particles using the QIAGEN RNeasy Micro Kit (QIAGEN, Hilden, Germany) following the manufacturer's protocol with an additional DNase digestion step on the column. Extracted RNA was reverse transcribed into cDNA prior qRT-PCR using the QuantiTect Reverse Transcription Kit (QIAGEN). For PCR, primers specific for wPRE, which is present on the construct, were used. Absolute copy numbers were calculated with the help of serial dilutions of a plasmid standard.

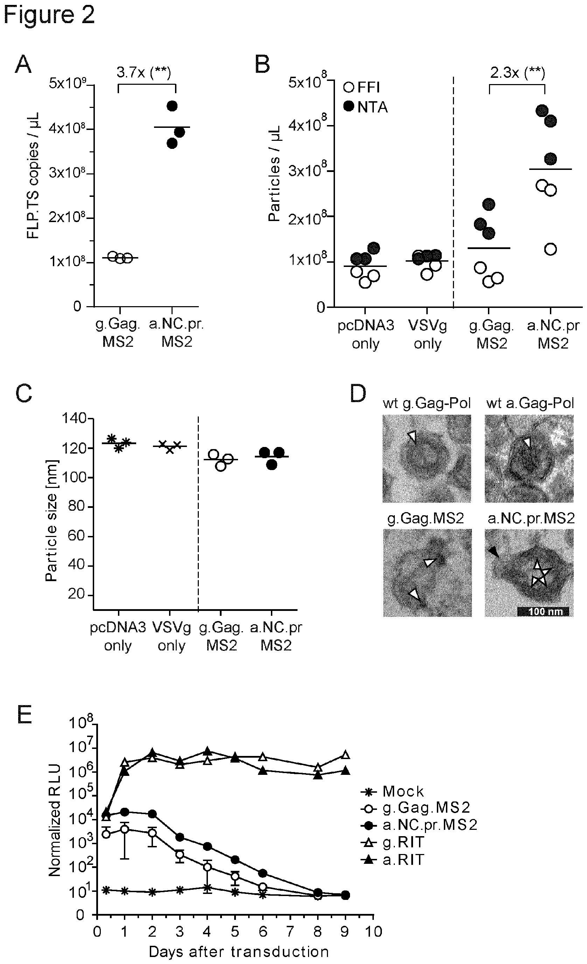

Fig. 2A shows the results of measuring copy numbers of mRNA encoding FLP recombinase in alpharetrovirus-based a.Gag.MS2 (a.NC.pr.MS2 variant) particle-based supernatants (•) according to the invention and in comparison the FLP recombinase mRNA content in gammaretrovirus-based g.Gag.MS2 supernatants (∘). Each data point reflects one independently prepared particle supernatant produced with the help of 293T producer cells. The results show higher concentrations of the gene of interest mRNA encoding FLP recombinase for supernatants containing the alpharetroviral Gag.MS2 particles.

Particle contents were determined in the same supernatants by full-field interferometry (FFI; (∘)) or by nanoparticle tracking analysis (NTA; (•)). As a further control, supernatants from 293T producer cells that were solely transfected with pcDNA3 or with an expression plasmid encoding VSVg were analyzed.

Fig. 2B shows the results of particle numbers for concentrated supernatants from producer cells that were only transfected with pcDNA3 (empty pcDNA3 plasmid) or the VSVg encoding plasmid (SEQ ID NO: 4). Both preparations served as controls for the estimation of the proportion of extracellular vesicles within alpha- and gammaretrovirus-based Gag.MS2 supernatants. In addition, gamma- and alpharetrovirus-based Gag.MS2 supernatants generated as described in Fig. 1A (g.Gag.MS2) or Fig. 1B (a.Gag.MS2, a.NC.pr.MS2, of SEQ ID NO: 1) were analyzed. The results show higher concentrations of virus-like particles consisting of the alpharetrovirus-based Gag.MS2 protein component.

Fig. 2C shows the results of viral particle size measurements of the same supernatants by NTA, revealing that the alpharetrovirus-based Gag.MS2 particles consisting of a.NC.pr.MS2 and delivering FLP.TS mRNA (•) show a similar size as the comparative gammaretrovirus-based particles (∘).

Fig. 2D shows transmission electron micrographs (TEM pictures, same enlargement, size bar is 100 nm) of alpharetrovirus-based Gag.MS2 particles (a.NC.pr.MS2) having the protein component MA-p2-p10-CA-NC-viral protease site-2xMS2CP and containing the mRNA encoding FLP recombinase with a 3'-terminal TS (FLP.TS) as also used in the constructs of Fig. 1C-E and Fig. 2A-C, and of comparative gammaretrovirus-based Gag.MS2 particles (g.Gag.MS2) containing the same mRNA construct. For comparison, wild-type viral vector particles having complete Gag-Pol are shown for alpharetrovirus- (wt a.Gag-Pol) and for gammaretrovirus- (wt g.Gag-Pol) derived vector particles. White arrowheads point to electron-dense capsids or parts of such. Black arrowheads highlight membrane protuberances that are visible in regions where the electron-dense capsids appear to be incomplete.

Fig. 2E shows results of determining Gag.MS2-mediated transgene expression, which is shown to be transient. The expression of firefly luciferase after transduction of HT1080 cells with firefly luciferase mRNA transferring Gag.MS2 particles was monitored over time. Respective alpha- and gammaretroviral integrating transfer vector particles (g.RIT and a.RIT) served as positive controls. In this experiment, the transient expression of firefly luciferase from the alpharetrovirus-based Gag.MS2 particles was found to be consistently higher than the transient expression from the gammaretrovirus-based Gag.MS2 particles. The comparative integrating constructs g.RIT and a.RIT show continuous high expression. Prior to this assay, the content of reporter mRNA (firefly luciferase) in supernatants was determined via qRT-PCR and volumes of supernatants were adjusted to apply equal amounts of firefly luciferase mRNA copies to target cells. Interestingly, in addition to the transient nature of both Gag.MS2 particle systems, a.Gag.MS2 particles consisting of a.NC.pr.MS2 show higher luciferase expression 1h after particle application, suggesting more functional particles per supernatant volume, more efficient cellular entry and/or other advantages during target cell transduction for a.Gag.MS2 particles.

### Example 2: Packaging and performance of alpharetrovirus-based a.Gag.MS2particles delivering CRISPR/Cas9 RNA (SpCas9 mRNA and respective sgRNAs)

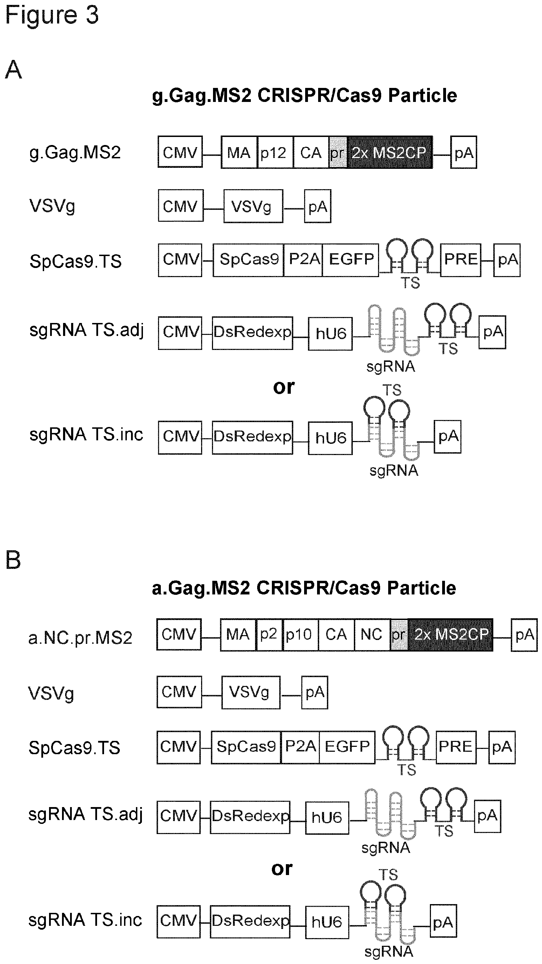

The components used for production of alpharetrovirus-based a.Gag.MS2 particles are schematically shown in Fig. 3B. The particles were produced in 293T producer cells that contained the expression cassettes as shown in Fig. 3B, an expression cassette encoding MAp2-p10-CA-NC-viral protease site-2xMS2CP (a.Gag.MS2, nucleotides 1462 to 3999 of SEQ ID NO: 1) under the control of the CMV promoter and a separate expression vector encoding VSVg under the control of the CMV promoter (SEQ ID NO: 4) for producing the mRNA of Streptococcus pyogenes Cas9 (SpCas9) that is fused to the coding sequence for the P2A protease site of the porcine teschovirus-1 followed by the coding sequence for EGFP, 2 copies of the MS2 target site hairpin (TS), a PRE element and a poly adenylation signal (pA). The latter construct is named SpCas9.TS (SEQ ID NO: 5). This construct encodes the mRNA for SpCas9-P2A-EGFP, linked to the TS, PRE and pA. From this construct, SpCas9 (gene of interest) and EGFP (reporter) are transcribed as a single mRNA transcript and are separated during translation via peptide bond skipping mediated by the P2A site.

The single guide RNAs (sgRNAs) sgRNA TS.adj or sgRNA TS.inc for co-operation with the SpCas9 enzyme in the target cell, are expressed with the help of the Pol III human polymerase promoter U6 (hU6) promoter, are linked to two MS2 TS copies and are terminated by a poly T stretch, preferably with a sequence TTTTT or TTTT. In addition, these constructs express DsRedexp under the control of the CMV promoter terminated by a poly adenylation signal (pA). The sgRNA TS.inc construct (SEQ ID NO: 3) has the TS copies incorporated within the sgRNA backbone, whereas in the sgRNA TS.adj (SEQ ID NO: 14) construct the TS copies were placed in 3' (adjacent) to the sgRNA backbone. Both sgRNA constructs were designed to allow easy replacement of respective protospacer sequences (without affecting the respective sgRNA.TS backbone), i.e. the part of the sgRNA that confers binding to the intended DNA target site within the recipient cell genome.

In these sgRNA constructs with the additional coding sequence for a GOI, represented by the reporter gene DsRedexp, the sgRNA is arranged between the GOI and the pA. These nucleic acid constructs contain the human codon-optimized coding sequence for DsRedexp (Discosoma sp. Red Fluorescent Protein, DsRed-Express, available from BD Clontech, product No. 6995-1) as a reporter gene for expression in a transfected target cell, whose coding sequence is linked to the promoter hU6 that in a transfected target cell controls transcription of the sgRNA.

The mRNA of the GOI, exemplified by DsRedexp, also encodes two copies of the MS2 hairpin structure (TS) at nucleotides 1681 to 1701 and at nucleotides 1751 to 1771 of SEQ ID NO: 3, which TS allow specific packaging of transcribed GOI.TS mRNA into MS2 coat protein dimer (2x MS2CP) of arising alpharetroviral particles. The protein components are encoded as the fusion protein within MA-p2-p10-CA-NC-viral protease site-2xMS2CP (a.Gag.MS2). This embodiment shows that a sgRNA, as an example of a short non-coding RNA, may be incorporated into the region of the TS, e.g. the sgRNA being arranged between two hairpin structures and/or immediately adjacent in 5' to the hairpin structures. As an example, SEQ ID NO: 3 includes a section, called BsmBI-stuffer at nucleotides 1560 to 1668, a fraction of which or which completely can be exchanged for the protospacer sequence of a sgRNA. Exemplary sgRNA sequences are given as SEQ ID NO: 7 for sgRNA for targeting mouse methylcytosine dioxygenase Tet2, SEQ ID NO: 8 for sgRNA targeting mouse cellular tumor antigen p53, SEQ ID NO: 9 for sgRNA for targeting human cellular tumor suppressor gene p53, SEQ ID NO: 10 for sgRNA for targeting human C-X-C chemokine receptor 4, SEQ ID NO: 11 for sgRNA for targeting enhanced green fluorescent protein (EGFP).

Fig. 3A schematically shows the components used for production of comparative gammaretrovirus-based Gag.MS2 particles that contain - in contrast to a.Gag.MS2 - the respective g.Gag.MS2 variant, the same pseudotyping protein VSVg and functionally identical RNA nucleic acid constructs.

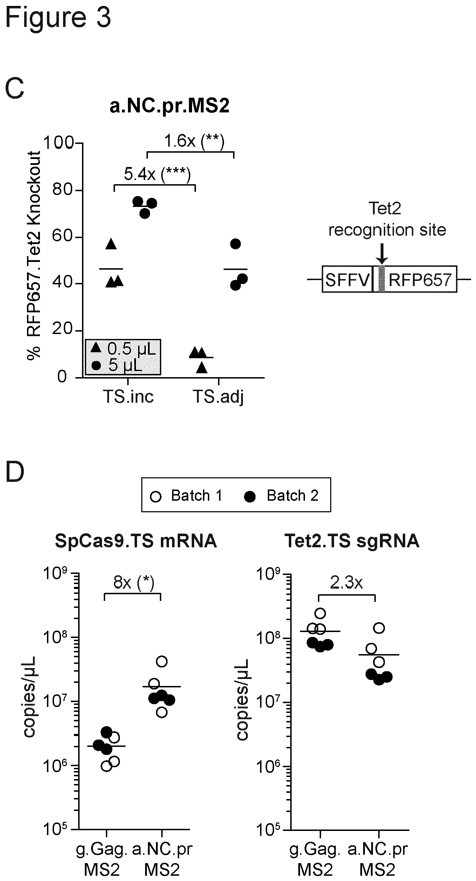

Fig. 3C shows that alpharetrovirus-based a.NC.pr.MS2 CRISPR/Cas9 particles that were packaged with TS.inc sgRNAs that are directed against murine *Tet methylcytosine dioxygenase* 2 gene (Tet2) perform better than their sgRNA.Tet.TS.adj correlates. Successful CRISPR/Cas9-mediated knockout of *RFP657.Tet2* (according to Heckl et al., loc cit.) in human HT1080 cells is indicated by the loss of RFP657 expression. Thus, a.NC.pr.MS2 CRISPR/Cas9 with sgRNA.TS.inc transcripts were used in the following figures. The reporter cassette is schematically shown, with the Tet2 recognition site arranged in the coding sequence for RFP657 (immediately downstream of the ATG start codon), so that CRISPR/Cas9 induced breaks of the DNA double-strand and an unprecise DNA repair result in a frame shift, and hence in loss of RFP657 expression. SFFV: Promoter from Spleen Focus Forming Virus.

Fig. 3D shows transcript numbers of mRNA contained in alpharetrovirus-based Gag.MS2 particles (a.Gag.MS2) and in comparative gammaretrovirus-based Gag.MS2 particles (g.Gag.MS2) for the SpCas9 encoding mRNA, linked to the TS, and for a murine Tet methylcytosine dioxygenase 2 gene (Tet2)-targeting sgRNA linked to the TS. The results show that the alpharetrovirus-based Gag.MS2 particles contain higher numbers of the SpCas9 mRNA, approx. 8-fold higher, but by a factor of approx. 2.3 lower numbers of the sgRNA, each compared to the gammaretrovirus-based Gag.MS2 particles. a.Gag.MS2 particles were packaged with Tet2.TS.inc, while g.Gag.MS2 particles were packaged with Tet2.TS.adj sgRNAs.

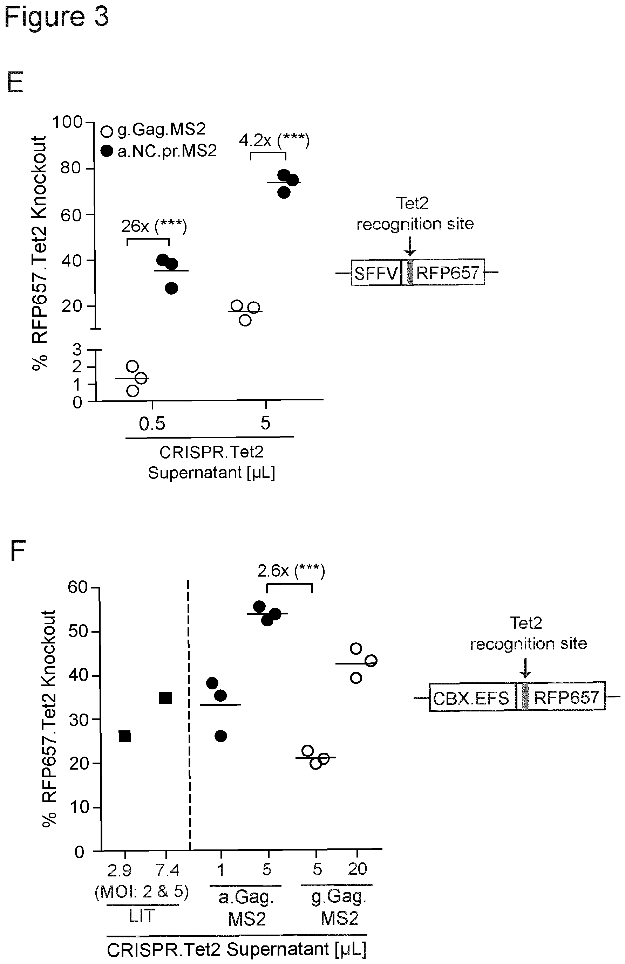

The activity of the SpCas9 enzyme in combination with the sgRNA in different target cells that were transduced with alpharetrovirus-based Gag.MS2 particles or comparative Gag.MS2 gammaretrovirus-based particles are shown in Fig. 3E, 3F.

The sgRNA is specific for the murine Tet methylcytosine dioxygenase 2 gene (Tet2) (protospacer see SEQ ID NO: 7), and in co-operation with the SpCas9 enzyme encoded by the mRNA of the virus-based particles will result in knockout of RFP657 in target cells, which contain a RFP657 reporter gene that has the corresponding Tet2 recognition site at its 5'-coding region (downstream of the ATG start codon) (SEQ ID NO: 7), also used in Fig. 3C. This reporter gene is expressed via the Spleen Focus Forming Virus (SFFV) promoter (Fig. 3C&E) or by the CBX.EFS promoter (epigenetic silencing resistant promoter consisting of the minimal ubiquitous chromatin-opening element CBX3 and the elongation factor 1 alpha short (EFS) promoter) (Fig. 3F).

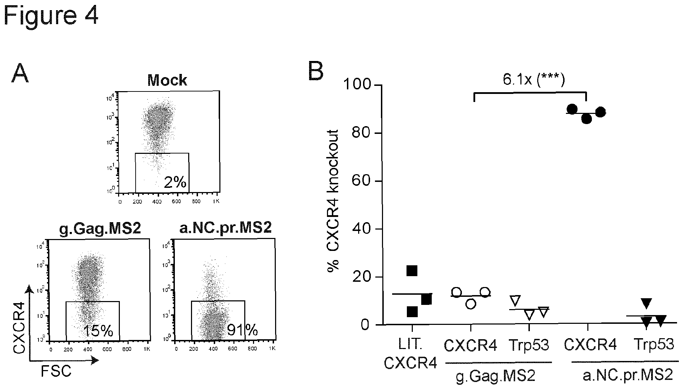

The superior knockout activity of the reporter gene RFP657 in human HT1080 fibroblasts (Fig. 3E) and in human iPSC (Fig. 3F) that contained the reporter cassette with the Tet2 recognition site or an endogenous *CXCR4* target gene in Jurkat cells (Example 3, Fig. 4) by an alpharetrovirus-based particle a.Gag.MS2 containing the nucleic acid constructs encoding CRISPR/Cas9 was found in comparison to otherwise similar gammaretrovirus-based particles. Successful CRISPR/Cas9-mediated knockout of *RFP657.Tet2* in target cells is indicated by the loss of RFP657 expression.

### Example 3: Packaging and performance of alpharetrovirus-based Gag.MS2particles delivering CRISPR/Cas9 RNA for manipulating an endogenous gene

As an example for an endogenous gene, the human CXCR4 gene was targeted by alpharetrovirus-based Gag.MS2 particles according to the invention.

Alpha- and gammaretrovirus-based Gag.MS2 particles were packaged with endogenous human CXCR4-targeting sgRNA.TS transcripts, containing the sgRNA including the protospacer sequence of SEQ ID NO: 10. Respective particles containing sgRNA.TS transcripts directed against the murine *Trp53* gene (corresponding protospacer shown in SEQ ID NO: 8) served as non-targeting negative controls. The results of Fig. 4A & B show that the alpharetrovirus-based particles are also more effective in manipulating a natural endogenous *CXCR4* target gene than gammaretrovirus-based particles. Interestingly, a.Gag.MS2 particles performed better than a lentiviral integrating CRISPR/Cas9 all-in-one vector control (LIT.CXCR4).

Fig. 4A shows representative FACS data of the knock-out of the endogenous CXCR4 gene in Jurkat cells. Untransduced cells served as Mock control. Alpha- and gammaretrovirus-based Gag.MS2 particles were packaged with CXCR4-targeting sgRNA.TS transcripts (g.Gag.MS2: CXCR4.TS.adj; a.Gag.MS2 CXCR4.TS.inc). Particles containing respective sgRNA.TS transcripts directed against the murine *Trp53* gene served as negative controls. The insets of Fig. 4A show that the knock-out of the endogenous CXCR4 gene targeted by the respective sgRNA.TS was only 15 % by the gammaretrovirus-based particles (g.Gag.MS2), but 91% for the alpharetrovirus-based particles (a.NC.pr.MS2) of the invention. Fig. 4B graphically shows this higher rate of genetic manipulation of the endogenous CXCR4 target gene by the alpharetrovirus-based particles.

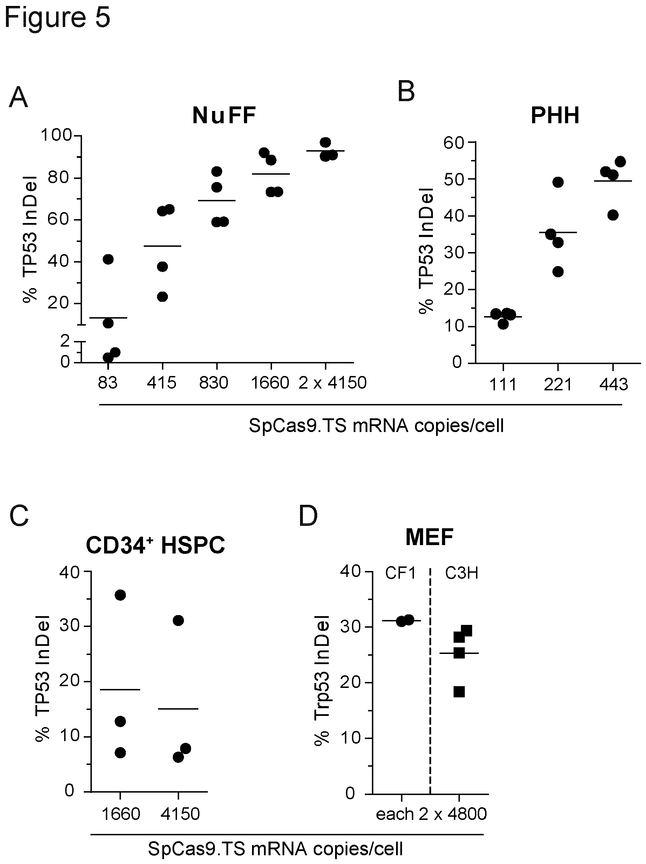

### Example 4: Performance of alpharetrovirus-based Gag.MS2 CRISPR/Cas9 particles targeting the endogenous human tumor suppressor gene TP53 in different primary human and murine cell types (Fig. 5)

The protein component of the alpharetrovirus-based Gag.MS2 particles was that described in preceding Examples. Respective alpharetrovirus-based Gag.MS2 particles contain the mRNA encoding SpCas9 followed by 2 copies of a TS hairpin (SpCas9.TS), optionally a PRE, and a polyA tail; and a separate sgRNA transcript specific for the human TP53 gene, with TS hairpins that are incorporated into the sgRNA backbone (TS.inc). The nucleic acid constructs are SEQ ID NO: 5 for the mRNA encoding SpCas9 nuclease, and SEQ ID NO: 9 for the protospacer replacing the BsmBI stuffer section of SEQ ID NO: 3.

The alpharetrovirus-based Gag.MS2 particles were produced in 293T producer cells, and particle containing supernatants were used for transducing target cells. Optionally, supernatants were concentrated by ultracentrifugation. The supernatants were analyzed by qRT PCR for the concentration of SpCas9.TS mRNA, and volumes of alpharetrovirus-based Gag.MS2 particle supernatants containing the copy numbers of the mRNA as indicated in the Figures were used for transducing human NuFF (Fig. 5A), primary human hepatocytes (PHH) (Fig. 5B), human cord blood-derived CD34+ hematopoietic stem and progenitor cells (CD34+ HSPC) (Fig. 5C), or murine embryonic fibroblasts (MEF; derived from CF1 or C3H mice) (Fig. 5D). The applied SpCas9.TS mRNA doses (copies/ cell) are shown on the X-axes, wherein 2x 4150 (see Fig. 5A) or 2x 4800 (see Fig. 5D) indicates two doses of alpharetrovirus-based a.Gag.MS2 particles, each having 4150 or 4800 mRNA copies, respectively. The Y-axes show the percentage of deletions in the target cells' TP53 gene.

These results show that the alpharetrovirus-based Gag.MS2 particles can effectively transduce human and murine primary cells, and that the encoded gene editing nuclease, e.g. SpCas9 in combination with a sgRNA, effectively reacts with the target cell genome. Further, these results show that the activity of the alpharetrovirus-based Gag.MS2 particles on target cells is dose-dependent.

### Example 5: Performance of alpharetrovirus-based Gag.MS2particles encoding CRISPR/Cas9 for simultaneously editing two or three genes/target sites in target cells

This example shows that the alpharetrovirus-based Gag.MS2 particles of the invention are effective in specifically manipulating, i.e. genetically engineering or simultaneously editing, two or three target genes of cells.

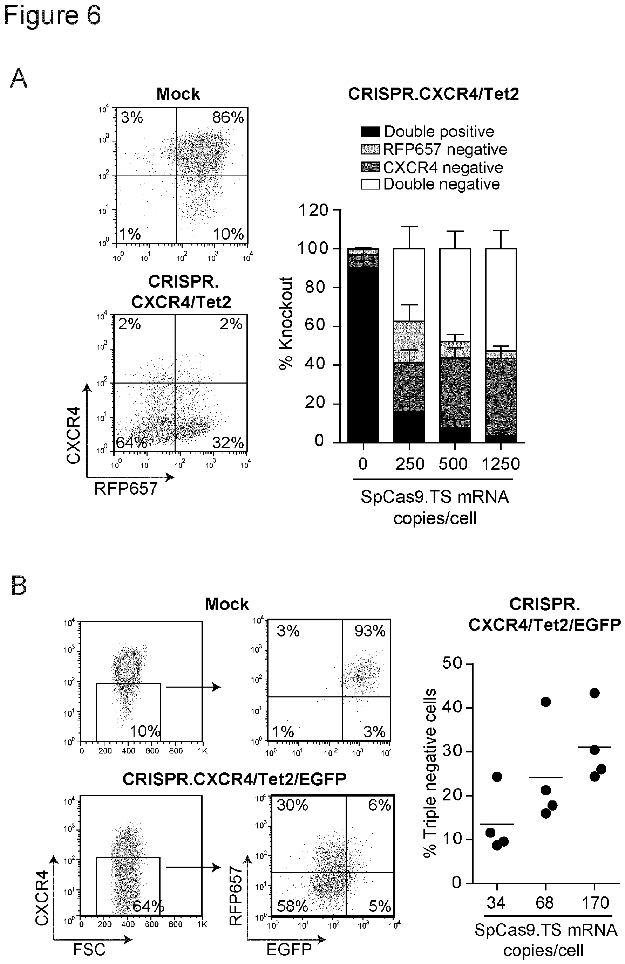

The protein component of the alpharetrovirus-based Gag.MS2 particles was that described in preceding Examples , with the packaged mRNA encoding SpCas9 mRNA linked upstream to a TS (SpCas9.TS, SEQ ID NO: 5) and the combination of a first sgRNA.TS.inc construct containing a protospacer sequence specific (SEQ ID NO: 10) for the human CXCR4 gene, with a second sgRNA.TS.inc construct containing a protospacer sequence specific (SEQ ID NO: 7) for the murine Tet2 gene, as an embodiment of alpharetrovirus-based Gag.MS2 particles containing nucleic acid constructs for editing two genes within target cells at the same time (Fig. 6A).

As a further embodiment, alpharetrovirus-based Gag.MS2 particles for simultaneously editing three genes / target sites in target cells. In addition to the SpCas9.TS.mRNA, alpharetrovirus-based particles containing a first sgRNA.TS.inc construct containing a protospacer sequence specific (SEQ ID NO: 10) for the human CXCR4 gene, with a second sgRNA.TS.inc construct containing a protospacer sequence specific for murine Tet2 gene (SEQ ID NO: 7), and a third sgRNA.TS.inc construct encoding a protospacer specific (SEQ ID NO: 11) for the EGFP gene were produced. As an alternative to or in addition to the sgRNA specific for knockout of a target gene or in addition to the SpCas9.TS.mRNA, an mRNA encoding a further GOI could be introduced by the alpharetrovirus-based particles.

Each embodiment consisted of the same protein components, namely the proteins according to the domains of alpharetroviral MA, p2, p10, CA, and NC, and linked via a linker to the NC which linker may contain a viral protease site, the MS2 coat protein dimer (2xMS2CP) expressed from SEQ ID NO: 1, and the VSVg expressed from SEQ ID NO: 4.

The target cells were Jurkat cells that endogenously express CXCR4, and that were transduced by lentiviral vectors (according to Hoffmann et al., loc cit, Heckl et al, loc cit, and Knopp et al., loc cit.) with nucleic acid constructs containing an expression cassette for EGFP and/or an expression cassette for the RFP657.Tet2 reporter gene.

Fig. 6A shows results for alpharetrovirus-based Gag.MS2 particles that contain the coding mRNA SpCas9.TS in combination with the sgRNA.TS.inc specific for CXCR4 and the sgRNA.TS.inc specific for Tet2. Untransduced/untreated cells served as a negative control (Mock). The representative FACS plots show that both the CXCR4 gene and the RFP657.Tet2 reporter gene (CXCR4/Tet2) were effectively knocked out by alpharetrovirus-based Gag.MS2 CRISPR/Cas9 particles. The columns representing the FACS results of three biological replicates show the numbers of % knockout in cells and demonstrate the dose dependency and positive correlation of alpharetrovirus-based Gag.MS2 particles, which are expressed as increasing SpCas9.TS mRNA copies per cell, with the amount of gene edited cells in treated cultures.

Fig. 6B shows FACS results of the Jurkat target cells after transduction with alpharetrovirus-based Gag.MS2 particles containing the mRNA encoding the gene editing nuclease SpCas9 in combination with additional three sgRNA.TS.inc transcripts, targeting human CXCR4, murine Tet2, and EGFP. Non-treated Mock-cells served as negative controls (Mock) and were highly positive for all three reporter genes, i.e. CXCR4, Tet2, EGFP, as depicted in the shown representative FACS plots. In contrast, target cells transduced with the alpharetrovirus-based Gag.MS2 particles showed effective knockout for all three targeted reporter genes. Cells that were to 64% negative for CXCR4 showed an additional knockout of 58% for RFP657 and EGFP reporter genes. The bar graph shows that increasing amounts of alpharetrovirus-based Gag.MS2 particles, determined as SpCas9.TS mRNA copies per target cell results in increasing proportions of triple-negative target cells that were genetically manipulated by SpCas9 and all three sgRNA.TS.inc transcripts, e.g. approx. 25% at 68 SpCas9.TS mRNA copies/cells, and approx. 30% at 170 SpCas9.TS mRNA copies/cell.

### Example 6: Transduction of self-amplifying RNA into cells by alpharetrovirus-based particles

As an embodiment of a further RNA, self-amplifying RNA replicons derived from Venezuelan equine encephalitis virus (VEE) were introduced into cells by alpharetrovirus-based particles of the invention. In this case, EGFP as a GOI was transferred. However, the transferred RNA may also be a non-coding RNA, e.g. a miRNA or IncRNA.

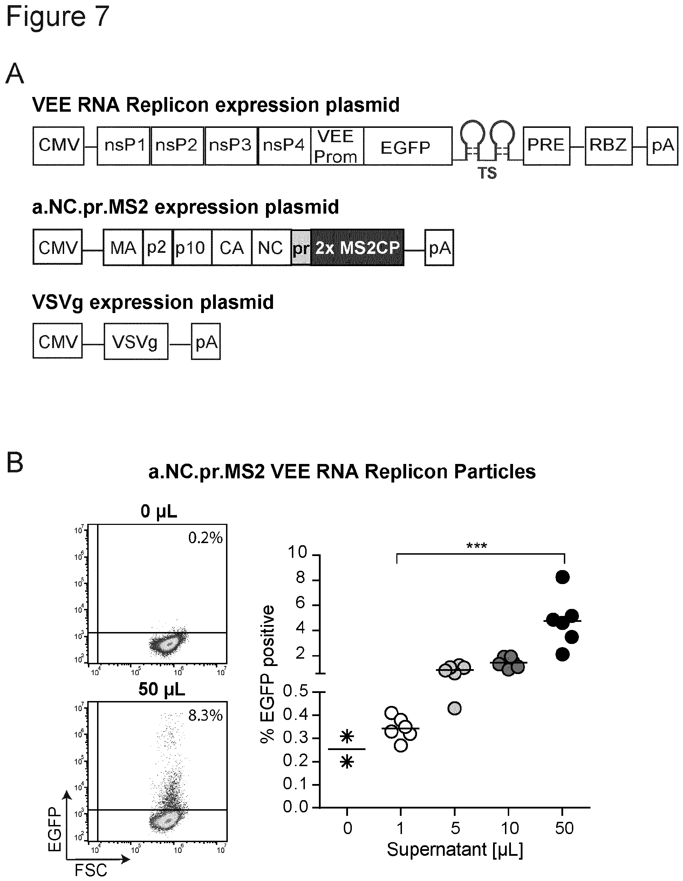

Fig. 7A schematically shows the nucleic acid constructs for alpharetrovirus-based a.Gag.MS2 particles (a.NC.pr.MS2)-transferring EGFP-encoding self-amplifying RNA replicons derived from Venezuelan equine encephalitis virus (VEE). On top, the expression plasmid for the generation of the VEE self-amplifying RNA replicon is shown. nsP1-4: VEE non-structural proteins; RBZ: HDV antigenomic ribozyme; VEE promoter: VEE subgenomic promoter. Fig. 7B shows representative FACS plots of HT1080 cells that were transduced with a.NC.pr.MS2-based VEE replicon particles. On the right, a summary of multiple biological replicates is shown. Increasing amounts of 100-fold concentrated supernatants resulted in increased percentages of EGFP-positive cells.

## Claims

1. Alpharetrovirus-based (Gag.MS2) particle comprising a protein component comprising alpharetroviral domains that consist of MA-p2-plO-CA-NC-PR, a linker of 7 to 25 amino acids and at least two MS2 coat proteins (2xMS2CP), a pseudotyping protein, and in association with the protein component at least one RNA construct encoding a gene of interest linked to at least one target site (TS).

2. Alpharetrovirus-based particle according to claim 1, **characterized in that** the protein component consists of MA-p2-p10-CA-NC.

3. Alpharetrovirus-based particle according to one of the preceding claims, **characterized in that** the alpharetroviral domains by hydrolysis of the linker are separated from the at least two MS2 coat proteins.

4. Alpharetrovirus-based particle according to one of the preceding claims, **characterized in that** between each of the alpharetroviral domains there is arranged a protease site.

5. Alpharetrovirus-based particle according to one of the preceding claims, **characterized in that** the linker between the alpharetroviral domains and the at least two MS2 domains is a viral protease site.

6. Alpharetrovirus-based particle according to one of the preceding claims, **characterized in that** the alpharetroviral domains have an amino acid sequence having at least 90% identity to the amino acid sequence encoded by nucleotides No. 1462 to 3573 of SEQ ID NO: 12 (MA-p2-p10-CA-NC-PR) or to the amino acid sequence encoded by nucleotides No. 1462 to 3186 of SEQ ID NO: 1,
the linker of 7 to 25 amino acids has an amino acid sequence having at least 90% identity to the amino acid sequence encoded by nucleotides No. 3187 to 3207 of SEQ ID NO: 1 or to the amino acid sequence encoded by nucleotides No. 3574 to 3594 of SEQ ID NO: 12, or is a glycin linker,
the at least two MS2 coat proteins comprise an amino acid sequence having at least 90% identity to the amino acid sequence encoded by nucleotides No. 3613 to 4389 of SEQ ID NO: 12 or encoded by nucleotides No. 3223 to 3999 of SEQ ID NO: 1.

7. Alpharetrovirus-based particle according to one of the preceding claims, **characterized in that** the RNA construct comprises a coding and/or a non-coding RNA or contains a self-amplifying RNA replicon, wherein the RNA construct is selected from single guide (sg) RNA linked to at least one target site (TS), short-hairpin (sh) RNA, micro RNA, shRNA/miRNA hybrid, self-amplifying RNA replicons, a coding sequence for a DNA recombinase, a coding sequence for an enzyme, a coding sequence for a receptor, a coding sequence for a transcription factor, a coding sequence for a gene editing nuclease, recombinase, transposon, a coding sequence for a for an antigen, or a combination of at least two of these.

8. Alpharetrovirus-based particle according to one of the preceding claims, **characterized in that** the RNA construct is a non-coding RNA to the 3' end of which a polyT stretch is linked.

9. Alpharetrovirus-based particle according to one of the preceding claims, **characterized by** comprising at least two different sgRNAs, each linked to at least one target site (TS).

10. Alpharetrovirus-based particle according to one of the preceding claims, **characterized in that** the RNA construct contains a sgRNA which is integrated with at least two target sites (TS) wherein the target sites are spaced by 60 to 40 nucleotides and the sgRNA is arranged between the hairpin sections of two TS domains, or the RNA construct contains a sgRNA which is arranged in 5' of directly adjacent at least two target sites (TS).

11. Alpharetrovirus-based particle according to one of the preceding claims, **characterized in that** the target site (TS) has a nucleotide sequence of at least 90%, preferably at least 95% to at least one sequence selected from nucleotides 1943 to 1965 and/or nucleotides 1982 to 2004 of SEQ ID NO: 2, of nucleotides 1681 to 1701 and/or nucleotides 1751 to 1771 of SEQ ID NO: 3, of nucleotides 1755 to 1775 and/or nucleotides 1794 to 1814 of SEQ ID NO: 14, of nucleotides 5697 to 5719 and/or nucleotides 5736 to 5758 of SEQ ID NO: 5, of nucleotides 2337 to 2359 and/or nucleotides 2376 to 2398 of SEQ ID NO: 6, and the 2xMS2CP has at least 90%, preferably at least 95% identity to an amino acid sequence encoded by nucleotides No. 3223 to 3999 of SEQ ID NO: 1.

12. Alpharetrovirus-based particle according to one of the preceding claims for use in medical treatment.

13. Alpharetrovirus-based particle according to one of the preceding claims, **characterized in that** the particle is pseudotyped with an envelope glycoprotein, which may target the particles to specific cell types and guide its target range.

14. Use of an alpharetrovirus-based particle according to one of the preceding claims for introducing at least one RNA construct into a cell.

15. Process for producing an alpharetrovirus-based particle according to one of claims 1 to 10 by expressing in a producer cell a protein component comprising or consisting of, from N-terminus to C-terminus alpharetroviral MA-p2-p10-CA-NC or MA-p2-p10-CA-NC-PR, a linker, and at least one MS2 coat protein dimer (2xMS2CP), and expressing a pseudotyping protein, and expressing at least one RNA construct encoding a gene of interest linked to at least one target site (TS).

16. Process according to claim 15, **characterized in that** the protein is proteolyzed at its protease site to separate the alpharetroviral domains from the at least one MS2 coat protein.
